# DEMANDE DE BREVET EUROPEEN

(11) **EP 2 277 863 A1**
(43) Date de publication de la demande: **26.01.2011**
(21) Numéro de dépôt: 10290410.9
(22) Date de dépôt: 20.07.2010
(51) Int. Cl.: C07D 211/42, A61K 31/445, A61K 31/4462, A61P 25/00

(54) **Composés pipéridiniques, leur procédé de préparation et les compositions pharmaceutiques qui les contiennent**

(30) Priorité: 21.07.2009 FR 0903573
(71) Demandeur: Les Laboratoires Servier, 92284 Suresnes Cedex (FR); Universite De Nantes I, 44035 Nantes Cedex 1 (FR)
(72) Inventeur: Botez, Luliana, 78800 Houilles (FR); Lebreton, Jacques, 44300 Nantes (FR); Lestage, Pierre, 78170 La Celle Saint Cloud (FR); Louis, Caroline, 75016 Paris (FR); Mathe, Monique, 44300 Nantes (FR); Caignard, Daniel-Henri, 95000 Boisemont (FR)

(57) **Abrégé**

Composés de formule (I) : 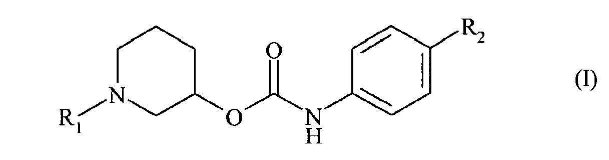 dans laquelle :
➢ R₁ représente un atome d'hydrogène ou un groupement méthyle,
➢ R₂ représente un atome de brome, un atome de fluor ou un groupement trifluorométhyle,

Médicaments.

## Description

La présente invention concerne de nouveaux composés pipéridiniques substitués, leur procédé de préparation, les compositions pharmaceutiques qui les contiennent ainsi que leur utilisation en tant que facilitateurs mnémocognitifs pour le traitement des troubles cognitifs associés aux pathologies du système nerveux central.

Le vieillissement de la population par augmentation de l'espérance de vie a entraîné parallèlement un large accroissement des troubles cognitifs liés au vieillissement cérébral normal ou au vieillissement cérébral pathologique survenant au cours de maladies neurodégénératives telles que, par exemple, la maladie d'Alzheimer.

La plupart des substances utilisées aujourd'hui pour le traitement des troubles cognitifs liés au vieillissement agissent en facilitant les systèmes cholinergiques centraux, soit directement comme c'est le cas des inhibiteurs de l'acétylcholinestérase (tacrine, donépézil) ou des agonistes cholinergiques (nefiracétam), soit indirectement comme dans le cas des nootropes (piracétam, pramiracétam) ou des vasodilatateurs cérébraux (vinpocétine).

Outre leurs propriétés cognitives, les substances agissant directement sur les systèmes cholinergiques centraux ont souvent des propriétés hypothermisantes qui peuvent être gênantes.

Il était donc particulièrement intéressant de synthétiser de nouveaux composés capables de s'opposer aux troubles cognitifs liés au vieillissement et/ou d'améliorer les processus cognitifs et dépourvus d'activité hypothermisante.

On connaît dans la littérature des composés pipéridiniques substitués décrits en tant que produits de synthèse et/ou d'alcaloïdes (J. Chem. Soc., Perkin Trans. 1, 1991, 3, pp. 611-616 ; Heterocycles, 1985, 23(4), pp. 831-834 ; Can. J. Chem., 1996, 74(12), pp. 2444-2453).

Les composés de la présente invention sont nouveaux et présentent des propriétés particulièrement intéressantes d'un point de vue pharmacologique.

La présente invention concerne plus particulièrement les composés de formule (I) : 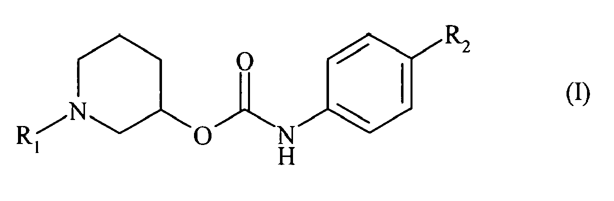 dans laquelle :
➢ R₁ représente un atome d'hydrogène ou un groupement méthyle,
➢ R₂ représente un atome de brome, un atome de fluor ou un groupement trifluorométhyle,
leurs énantiomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

Le groupement R₁ représente avantageusement un groupement méthyle.

Le groupement R₂ préféré est l'atome de brome.

L'invention concerne préférentiellement les composés qui sont :
- le (4-bromophényl)carbamate de 1-méthyl-pipéridin-3-yle,
- le (4-bromophényl)carbamate de (3*S*)-1-méthyl-pipéridin-3-yle,
- le (4-bromophényl)carbamate de (3*R*)-1-méthyl-pipéridin-3-yle,

Les sels d'addition à un acide ou une base pharmaceutiquement acceptable des composés préférés de l'invention font partie intégrante de l'invention.

L'invention s'étend également au procédé de préparation des composés de formule (I) caractérisé en ce que l'on utilise comme produit de départ le composé de formule (II) : 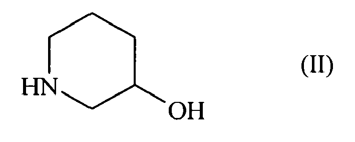 qui subit une étape de protection sur l'atome d'azote pour donner le composé de formule (III) : 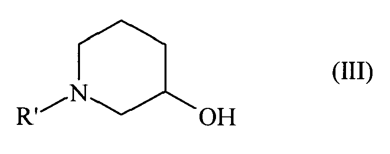 dans laquelle R' représente un groupement protecteur de la fonction amine, comme par exemple le groupement *tert*-butyloxycarbonyle,
qui est ensuite soumis à l'action d'un dérivé de formule (IV), 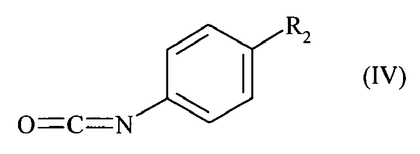 dans laquelle R₂ est tel que défini dans la formule (I),
pour conduire au composé de formule (V) : 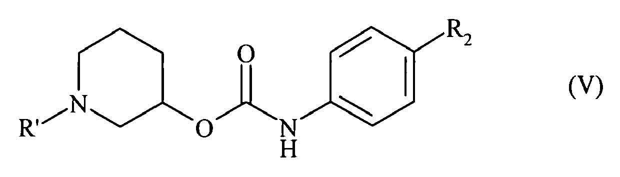 dans laquelle R₂ et R' sont tels que défini précédemment,
qui est ensuite soumis à une réaction de déprotection de la fonction amine, par exemple en présence d'acide trifluoroacétique, pour conduire aux composés de formule (I/a), cas particulier des composés de formule (I), 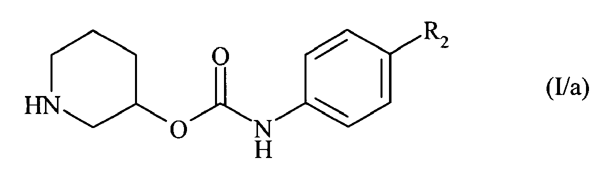 dans laquelle R₂ est tel que défini précédemment,
qui peut éventuellement subir une réaction de méthylation pour conduire aux composés de formule (I/b), cas particulier des composés de formule (I), 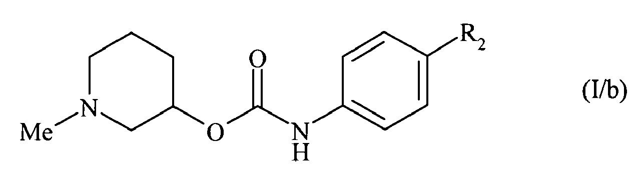 dans laquelle R₂ est tel que défini précédemment,
une variante dans la préparation du composé de formule (I/b) consistant en l'utilisation comme produit de départ le composé de formule (VI) : 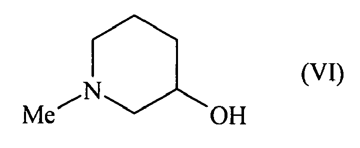 qui est soumis à l'action d'un dérivé de formule (IV),
les composés de formule (I/a) et (I/b), qui constituent l'ensemble des composés de formule (I), peuvent être ensuite purifiés selon une technique classique de séparation, que l'on transforme, si on le souhaite, en ses sels d'addition à un acide ou à une base pharmaceutiquement acceptable et dont les énantiomères peuvent être séparés sur colonne chirale, selon une technique classique de séparation.

Les composés de formule (II), (IV) et (VI) sont commerciaux ou aisément accessibles à l'homme du métier par des réactions de chimie classiques ou décrites dans la littérature.

Les composés de la présente invention, outre le fait qu'ils soient nouveaux, présentent des propriétés facilitatrices des processus cognitifs qui les rendent utiles dans le traitement des déficits cognitifs associés au vieillissement cérébral et aux maladies neurodégénératives telles que la maladie d'Alzheimer, la maladie de Parkinson, la maladie de Pick, la maladie de Korsakoff, les démences vasculaires, les démences frontales et sous-corticales ainsi que la schizophrénie.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc. La posologie utile est adaptable selon la nature et la sévérité de l'affection, la voie d'administration ainsi que l'âge et le poids du patient. Cette posologie varie de 0,01 mg à 1 g par jour en une ou plusieurs prises.

Les exemples suivants illustrent l'invention mais ne la limitent en aucune façon.

Les structures des composés décrits dans les exemples ont été déterminées selon les techniques spectrophotométriques usuelles (infrarouge, RMN, spectrométrie de masse).

### EXEMPLE 1 : (4-Bromophényl)carbamate de 1-méthylpipéridin-3-yle

A une solution de 3-hydroxy-1-méthylpipéridine (21,2 mmol) en agitation dans du toluène anhydre (50 mL) et sous atmosphère d'argon, est additionné l'isocyanate de *para*-bromophényle (25,4 mmol) à température ambiante. Le mélange réactionnel est agité pendant 24 heures à 60 °C. Après filtration sur fritté, le solide est lavé avec du dichlorométhane. Le filtrat est évaporé à sec sous pression réduite puis repris avec du dichlorométhane. La phase organique est lavée par une solution aqueuse saturée en NaHCO₃, séchée sur MgSO₄, filtrée puis évaporée sous pression réduite. Le solide jaune obtenu est trituré dans de l'éther diisopropylique (60 ml). Après filtration, le produit du titre est obtenu sous forme d'une poudre blanche.

### Point du fusion : 127 °C

### EXEMPLE 2 : (4-Bromophényl)carbamate de (3R)-1-méthylpipéridin-3-yle

L'énantiomère *R* a été obtenu par séparation du mélange racémique obtenu dans l'Exemple 1. La séparation a été effectuée sur colonne Chiralpak AS à température ambiante avec une détection UV à 275 nm, en utilisant comme éluant une mélange acétonitrile / diéthylamine 100 / 0,1.
*Pureté optique : > 99 %*
*Pouvoir rotatoire : [*α*] ^{20°C} ₅₈₉ₙₘ* = + *13,57° (c = 0, 6 MeOH)*

| *Microanalyse élémentaire :* | | | | |
|---|---|---|---|---|
| | *C* | *H* | *N* | *Br* |
| *% théorique* | *49,86* | *5,47* | *8,94* | *25,51* |
| *% expérimental* | *49,79* | *5,48* | *9, 22* | *26,30* |

### EXEMPLE 3 : (4-Bromophényl)carbamate de (3S)-1-méthylpipéridin-3-yle

L'obtention de l'énantiomère *S* a été effectuée selon les mêmes conditions décrites dans l'Exemple 2 à partir du mélange racémique obtenu dans l'Exemple 1.
*Pureté optique : > 99 %*
*Pouvoir rotatoire : [*α*] ^{20°C} ₅₈₉ₙₘ* = - *13,39° (c = 1,0 MeOH)*

| *Microanalyse élémentaire :* | | | | |
|---|---|---|---|---|
| | *C* | *H* | *N* | *Br* |
| *% théorique* | *49,86* | *5,47* | *8,94* | *25,51* |
| *% expérimental* | *49,96* | *5,50* | *9, 07* | *25,98* |

### EXEMPLE 4 : (4-Fluorophényl)carbamate de 1-méthylpipéridin-3-yle

A une solution de 3-hydroxy-1-méthylpipéridine (34,7 mmol) en agitation dans du toluène anhydre (50 mL) et sous atmosphère d'argon, est additionné l'isocyanate de *para*-fluorophényle (44,0 mmol) à température ambiante. Le mélange réactionnel est agité pendant 48 heures à 60 °C. Une fois filtré sur fritté, le solide est lavé avec du dichlorométhane. Le filtrat est évaporé à sec sous pression réduite puis repris avec du dichlorométhane. La phase organique est lavée par une solution aqueuse saturée en NaHCO₃, séchée sur MgSO₄, filtrée puis évaporée sous pression réduite. L'huile orange obtenue est purifiée par chromatographie sur gel de silice avec comme éluant un mélange dichlorométhane / méthanol (gradient de 99 / 1 à 95 / 5). Le solide blanc obtenu est recristallisé dans un minimum d'éther diisopropylique, pour donner le produit du titre sous forme d'une poudre blanche.
*Point du fusion : 97-98 °C*

### EXEMPLE 5 : (4-Fluorophényl)carbamate de (3R)-1-méthylpipéridin-3-yle

L'énantiomère *R* a été obtenu par séparation sur colonne préparative chirale.

| *Microanalyse élémentaire :* | | | |
|---|---|---|---|
| | *C* | *H* | *N* |
| *% théorique* | *61,89* | *6,79* | *11,10* |
| *% expérimental* | *62, 06* | *6,98* | *10,96* |

### EXEMPLE 6 : (4-Fluorophényl)carbamate de (3S)-1-méthylpipéridin-3-yle

L'énantiomère *S* a été obtenu par séparation sur colonne préparative chirale.

### EXEMPLE 7 : (4-Trifluorométhylphényl)carbamate de 1-méthylpipéridin-3-yle

A une solution de 3-hydroxy-1-méthylpipéridine (4,8 mmol) en agitation dans du toluène anhydre (10 mL) et sous atmosphère d'argon, est additionné l'isocyanate de *para*-trifluorométhylphényle (7 mmol) à température ambiante. Le mélange réactionnel est agité pendant 17 heures à 55 °C. Le brut réactionnel est filtré sur fritté puis le solide est lavé avec du dichlorométhane. Le filtrat est évaporé à sec sous pression réduite puis repris avec du dichlorométhane. La phase organique est lavée par une solution aqueuse saturée en NaHCO₃, séchée sur MgSO₄, filtrée puis évaporée sous pression réduite. Le solide est purifié par chromatographie sur colonne de gel de silice avec comme éluant un mélange dichlorométhane / méthanol (gradient de 98 / 2 à 95 / 5) pour donner le produit du titre sous forme d'une poudre blanche.

### Point du fusion : 119-120 ° C

### EXEMPLE 8 : (4-Trifluorométhylphényl)carbamate de (3S)-1-méthylpipéridin-3-yle

L'énantiomère *S* a été obtenu par séparation sur colonne préparative chirale.
*Pouvoir rotatoire : [*α*] ^{20°C} ₅₈₉ₙₘ* = - *12,44° (c = 0, 6 MeOH)*

| *Microanalyse élémentaire :* | | | |
|---|---|---|---|
| | *C* | *H* | *N* |
| *% théorique* | *55, 63* | *5, 67* | *9, 27* |
| *% expérimental* | *55,45* | *6, 04* | *9, 26* |

### EXEMPLE 9 : 4-(Trifluorométhylphényl)carbamate de (3R)-1-méthylpipéridin-3-yle

L'énantiomère *R* a été obtenu par séparation sur colonne préparative chirale.
*Pouvoir-rotatoire : [*α*] ^{20°C} ₅₈₉ₙₘ* = + *13,96° (c = 0,7 MeOH)*

| *Microanalyse élémentaire :* | | | |
|---|---|---|---|
| | *C* | *H* | *N* |
| *% théorique* | *55, 63* | *5,67* | *9, 27* |
| *% expérimental* | *55, 67* | *6, 26* | *9,32* |

### EXEMPLE 10 : (4-Trifluorométhylphényl)carbamate de pipéridin-3-yle

### Stade A : 3-Hydroxypipéridine-1-carboxylate de tert-butyle

A une solution de 3-hydroxypipéridine (39,6 mmol) en agitation dans l'eau (200 mL), sont additionnés du NaHCO₃ (119 mmol) puis le dicarbonate de di-*tert*-butyle (47,5 mmol) à température ambiante. Le mélange réactionnel est agité pendant 3 jours à température ambiante puis la phase aqueuse est extraite trois fois par du dichlorométhane. Les phases organiques sont réunies, séchées sur MgSO₄, filtrées puis évaporées sous pression réduite. L'huile incolore obtenue est laissée à l'air libre et à température ambiante pendant 4 jours. Les cristaux formés sont alors broyés en poudre puis mis sous pompe à palette pendant 24 heures. Le produit du titre est obtenu sous forme de cristaux blancs sans purification supplémentaire.

### Stade B : 3-{[(4-Trifluorométhylphényl)amino]carbonyl}oxypipéridine-1-carboxylate de tert-butyle

A une solution du composé obtenu au Stade précédent (8,4 mmol) en agitation dans du toluène anhydre (30 mL) et sous atmosphère d'argon, est additionné l'isocyanate de *para*-trifluorométhylphényle (13,3 mmol) à température ambiante. Le mélange réactionnel est agité pendant 48 heures à 55 °C. Après filtration sur fritté, le solide est lavé abondamment avec du dichlorométhane. Le filtrat est évaporé à sec sous pression réduite pour donner le produit du titre sous forme d'une poudre blanche.

### Stade C : (4-Trifluorométhylphényl)carbamate de pipéridin-3-yle

A une solution du composé obtenu au Stade précédent (6,9 mmol) en agitation dans du dichlorométhane anhydre (24 mL) et sous atmosphère d'argon, est additionné l'acide trifluoroacétique (10,2 ml) dans un bain de glace. Le mélange réactionnel est agité pendant 1 heure à température ambiante. Le brut réactionnel est versé dans une solution aqueuse de soude 2 M (100 ml) refroidie par un bain de glace. La phase aqueuse basique est extraite deux fois par du dichlorométhane. Les phases organiques sont réunies, lavées par une solution aqueuse saturée en NaCl, séchées sur MgSO₄, filtrées puis évaporées sous pression réduite pour conduire au produit du titre sous forme d'une poudre.

### Point du fusion : 150-151 °C

### EXEMPLE 11 : (4-Trifluorométhylphényl)carbamate de (3S)-pipéridin-3-yle

L'énantiomère *S* a été obtenu par séparation du mélange racémique obtenu dans l'Exemple 10. La séparation a été effectuée sur colonne Chiralpak AD à température ambiante avec une détection UV à 245 nm, en utilisant comme éluant un mélange acétonitrile / diéthylamine 100 / 0,1.
*Pureté optique : > 99* %
*Pouvoir rotatoire : [*α*] ^{20°C} ₅₈₉ₙₘ* = - *14,04° (c = 0, 9 MeOH)*

| *Microanalyse élémentaire :* | | | |
|---|---|---|---|
| | *C* | *H* | *N* |
| *% théorique* | *54,17* | *5, 24* | *9,72* |
| *% expérimental* | *54, 20* | *5,38* | *9, 62* |

### EXEMPLE 12 : (4-Trifluorométhylphényl)carbamate de (3R)-pipéridin-3-yle

L'obtention de l'énantiomère *R* a été effectuée selon les mêmes conditions décrites dans l'Exemple 11 à partir du mélange racémique obtenu dans l'Exemple 10.
*Pureté optique : 99 %*
*Pouvoir rotatoire : [α] ^{20°C} ₅₈₉ₙₘ* = + *13,43° (c = 1,2 MeOH)*

| *Microanalyse élémentaire :* | | | |
|---|---|---|---|
| | *C* | *H* | *N* |
| *% théorique* | *54,17* | *5, 24* | *9,72* |
| *% expérimental* | *54, 08* | *5,44* | *9, 61* |

### EXEMPLE 13 : (4-Bromophényl)carbamate de pipéridin-3-yle

### Stade A : 3-{[(4-Bromophényl)amino]carbonyl}oxypipéridine-1-carboxylate de tert-butyle

A une solution du composé obtenu au Stade A de l'Exemple 10 (2,49 mmol) en agitation dans du toluène anhydre (10 mL) et sous atmosphère d'argon, est additionné l'isocyanate de *para*-bromophényle (5,05 mmol) à température ambiante. Le mélange réactionnel est agité pendant 72 heures à 60 °C. Le brut réactionnel est filtré sur fritté, évaporé à sec sous pression réduite puis purifié par chromatographie sur gel de silice avec comme éluant un mélange dichlorométhane / éther diéthylique (gradient de 100 / 0 à 90 / 10). Le produit du titre est obtenu sous forme d'une poudre blanche.

### Stade B : (4-Bromophényl)carbamate de pipéridin-3-yle

A une solution du composé du Stade précédent (10 mmol) en agitation dans du dichlorométhane anhydre (50 mL) et sous atmosphère d'argon, est additionné l'acide trifluoroacétique (13 ml) dans un bain de glace. Le mélange réactionnel est agité pendant 2 heures à température ambiante. Le brut réactionnel est versé sur une solution saturée en K₂CO₃ à 0 °C. La phase aqueuse est extraite deux fois par du dichlorométhane. Les phases organiques sont réunies, lavées par une solution de soude 2 N puis par une solution saturée de NaCl, séchées sur MgSO₄, filtrées puis évaporées pour donner le produit du titre sous forme d'un solide blanc.
*Point du fusion : 169-170 °C*

### EXEMPLE 14 : (4-Bromophényl)carbamate de (3S)-pipéridin-3-yle

L'énantiomère *S* a été obtenu par séparation sur colonne préparative chirale.

| *Microanalyse élémentaire :* | | | | |
|---|---|---|---|---|
| | *C* | *H* | *N* | *Br* |
| *% théorique* | *48,18* | *5,05* | *9,36* | *26,71* |
| *% expérimental* | *48,86* | *5,14* | *8,93* | *25,35* |

### EXEMPLE 15 : (4-Bromophényl)carbamate de (3R)-pipéridin-3-yle

### L'énantiomère R a été obtenu par séparation sur colonne préparative chirale.

### EXEMPLE 16 : (4-Fluorophényl)carbamate de pipéridin-3-yle

### Stade A : 3-{[(4-Fluorophenyl)amino]carbonyl}oxypipéridine-1-carboxylate de tert-butyle

A une solution du composé obtenu au Stade A de l'Exemple 10 (15 mmol) en agitation dans du toluène anhydre (50 mL) et sous atmosphère d'argon, est additionné l'isocyanate de *para*-fluorophényle (18 mmol) à température ambiante. Le mélange réactionnel est agité pendant 72 heures à 60 °C. Une fois filtré sur fritté, le solide est repris avec du dichlorométhane (40 ml), puis filtré à nouveau sur fritté. La phase organique est lavée par une solution aqueuse saturée en NaHCO₃, séchée sur MgSO₄, filtrée puis évaporée sous pression réduite. Le produit du titre est obtenu sous forme d'une poudre blanche.

### Stade B : (4-Fluorophényl)carbamate de pipéridin-3-yle

A une solution du composé obtenu au Stade précédent (18,4 mmol) en agitation dans du dichlorométhane anhydre (100 mL) et sous atmosphère d'argon, est additionné l'acide trifluoroacétique (23 ml) dans un bain de glace. Le mélange réactionnel est agité pendant 3 heures à température ambiante. Le brut réactionnel est versé sur une solution aqueuse de soude 2 M (100 ml) placée dans un bain de glace. La phase aqueuse est extraite deux fois par du dichlorométhane. Les phases organiques sont réunies, lavées par une solution aqueuse saturée en NaCl, séchées sur MgSO₄, filtrées puis évaporées sous pression réduite. Le brut est purifié par chromatographie sur colonne d'alumine avec comme éluant un mélange dichlorométhane / méthanol (gradient de 98 / 2 à 95 / 5) pour donner le produit du titre sous forme d'une poudre blanche.
*Point du fusion : 147-148 °C*

### EXEMPLE 17 : (4-Fluorophényl)carbamate de (3R)-pipéridin-3-yle

L'énantiomère *R* a été obtenu par séparation sur colonne préparative chirale.

### EXEMPLE 18 : (4-Fluorophényl)carbamate de (3S)-pipéridin-3-yle

L'énantiomère *S* a été obtenu par séparation sur colonne préparative chirale.

### ETUDE PHARMACOLOGIQUE

### EXEMPLE A : Etude de la toxicité aiguë et des effets hypothermisants

La toxicité aiguë a été appréciée après administration orale à des lots de 5 souris (26 ± 2 grammes). Les animaux ont été observés à intervalles réguliers au cours de la première journée et quotidiennement pendant les deux semaines suivant le traitement.

Les résultats montrent que les composés de la présente invention n'entraînent pas d'effets indésirables ni de modifications de la température jusqu'à la dose maximale testée de 100 mg/kg *per os.*

### EXEMPLE B : Reconnaissance sociale chez le rat Wistar

Initialement décrit en 1982 par Thor et Holloway (J. Comp. Physiol., 1982, 96, 1000-1006), le test de la reconnaissance sociale a été ensuite proposé par différents auteurs (Dantzer et al, Psychopharmacology, 1987, 91, 363-368 ; Perio et al, Psychopharmacology, 1989, 97, 262-268) pour l'étude des effets mnémocognitifs de nouveaux composés. Fondé sur l'expression naturelle de la mémoire olfactive du rat et sur son oubli naturel, ce test permet d'apprécier la mémorisation, par la reconnaissance d'un jeune congénère, par un rat adulte. Un jeune rat (21 jours), pris au hasard, est placé dans la cage de stabulation d'un rat adulte pendant 5 minutes. Par l'intermédiaire d'un dispositif vidéo, l'expérimentateur observe le comportement de reconnaissance sociale du rat adulte et en mesure la durée globale. Puis le jeune rat est ôté de la cage du rat adulte et est placé dans une cage individuelle, jusqu'à la seconde présentation. Le rat adulte reçoit alors le produit à tester et, 2 heures plus tard, est remis en présence (5 minutes) du jeune rat. Le comportement de reconnaissance sociale est alors à nouveau observé et la durée en est mesurée. Le critère de jugement est la différence (T₂-T₁), exprimée en secondes, des temps de "reconnaissance" des 2 rencontres.

Les résultats obtenus montrent une différence (T₂-T₁) comprise entre (-19) et (-40) secondes pour des doses allant de 0,3 à 3 mg/kg. Ces résultats démontrent une augmentation très importante, et à faible dose, de la mémorisation.

### EXEMPLE C : Reconnaissance d'objet chez le rat Wistar

Le test de reconnaissance d'objet chez le rat Wistar a été initialement développé par Ennaceur et Delacour (Behav. Brain Res., 1988, 31, 47-59). Ce test est basé sur l'activité exploratoire spontanée de l'animal et possède les caractéristiques de la mémoire épisodique chez l'homme. Sensible au vieillissement (Scali et al, Eur. J. Pharmacol., 1997, 325, 173-180), ainsi qu'aux dysfonctionnements cholinergiques (Bartolini et al, Pharm. Biochem. Behav. 1996, 53(2), 277-283), ce test de mémoire est fondé sur l'exploration différentielle de 2 objets de forme assez similaire, l'un familier, l'autre nouveau. Préalablement au test, les animaux sont habitués à l'environnement (enceinte sans objet). Au cours d'une 1^{ère} session, les rats sont placés (3 minutes) dans l'enceinte où se trouvent 2 objets identiques. La durée d'exploration de chaque objet est mesurée. Au cours de la 2^{e} session (3 minutes), 24 heures plus tard, 1 des 2 objets est remplacé par un nouvel objet. La durée d'exploration de chaque objet est mesurée. Le critère de jugement est la différence Delta, exprimée en seconde, des temps d'exploration de l'objet nouveau et de l'objet familier au cours de la 2^{e} session. Les animaux témoins, traités préalablement au véhicule par voie *per os* 60 minutes avant chaque session, explorent de façon identique l'objet familier et l'objet nouveau, ce qui signe l'oubli de l'objet déjà présenté. Les animaux traités par un composé facilitateur mnémocognitif, explorent de façon préférentielle l'objet nouveau, ce qui signe le souvenir de l'objet déjà présenté.

Les résultats obtenus montrent une différence Delta comprise entre 6 et 12 secondes, pour des doses allant de 0,3 à 3 mg/kg. Ces résultats démontrent une augmentation importante, et à très faible dose, de la mémorisation.

### EXEMPLE D : Composition pharmaceutique

| Formule de préparation pour 1000 comprimés dosés à 10 mg en (4-bromophényl) |
|---|
| carbamate de (3*S*)-1-méthyl-pipéridin-3-yle (Exemple 3) ............................................... 10 g |
| Hydroxypropylcellulose ..... 2 g |
| Amidon de blé ..... 10 g |
| Lactose..... 100 g |
| Stéarate de magnésium ..... 3 g |
| Talc ..... 3 g |

## Revendications

1. Composés de formule (I) : 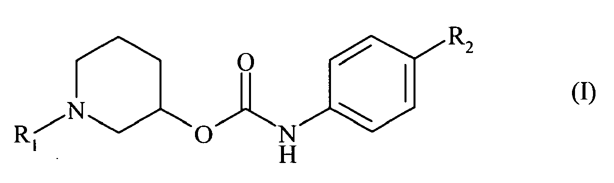 dans laquelle :
➢ R₁ représente un atome d'hydrogène ou un groupement méthyle,
➢ R₂ représente un atome de brome, un atome de fluor ou un groupement trifluorométhyle,
leurs énantiomères, ainsi que leurs sels d'addition à un acide ou une base pharmaceutiquement acceptable.

2. Composés de formule (I) selon la revendication 1, **caractérisés en ce que** le groupement R₁ représente un méthyle.

3. Composés de formule (I) selon la revendication 1 ou 2, **caractérisés en ce que** le groupement R₂ représente un atome de brome.

4. Composé de formule (I) selon la revendication 1 qui est le (4-bromophényl)carbamate de 1-méthyl-pipéridin-3-yle, ses énantiomères ainsi que ses sels d'addition à un acide ou une base pharmaceutiquement acceptable.

5. Composé de formule (I) selon la revendication 1 qui est le (4-bromophényl)carbamate de (3*S*)-1-méthyl-pipéridin-3-yle, ainsi que ses sels d'addition à un acide ou une base pharmaceutiquement acceptable.

6. Composé de formule (I) selon la revendication 1 qui est le (4-bromophényl)carbamate de (3*R*)-1-méthyl-pipéridin-3-yle, ainsi que ses sels d'addition à un acide ou une base pharmaceutiquement acceptable.

7. Procédé de préparation des composés de formule (I) selon la revendication 1, **caractérisé en ce que** l'on utilise comme produit de départ un composé de formule (II) : 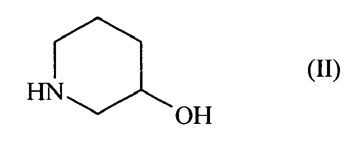 qui subit une étape de protection sur l'atome d'azote pour donner le composé de formule (III) : 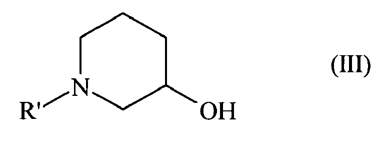 dans laquelle R' représente un groupement protecteur de la fonction amine, comme par exemple le groupement tert-butyloxycarbonyle,
qui est ensuite soumis à l'action d'un dérivé de formule (IV), 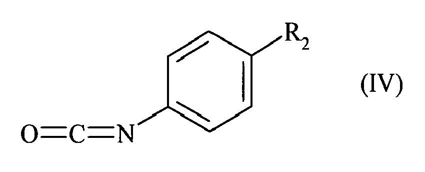 dans laquelle R₂ est tel que défini dans la revendication 1,
pour conduire au composé de formule (V) : 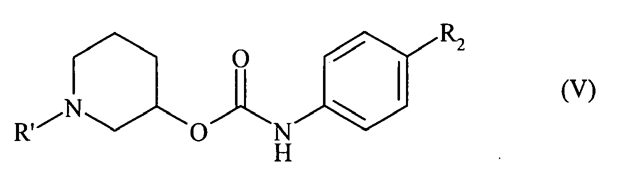 dans laquelle R₂ et R' sont tels que défini précédemment,
qui est ensuite soumis à une réaction de déprotection de la fonction amine, par exemple en présence d'acide trifluoroacétique, pour conduire aux composés de formule (I/a), cas particulier des composés de formule (I), 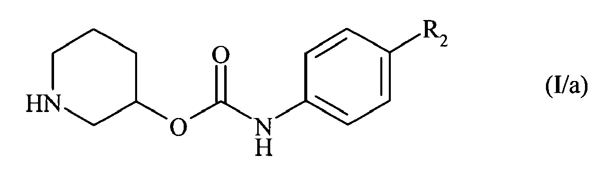 dans laquelle R₂ est tel que défini précédemment,
qui peut éventuellement subir une réaction de méthylation pour conduire aux composés de formule (I/b), cas particulier des composés de formule (I), 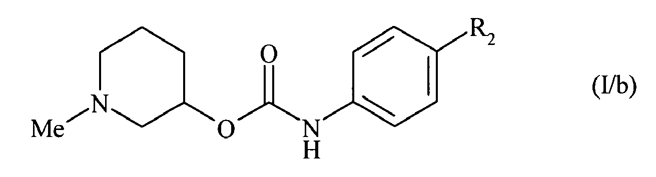 dans laquelle R₂ est tel que défini précédemment,
une variante dans la préparation du composé de formule (I/b) consistant en l'utilisation comme produit de départ le composé de formule (VI) : 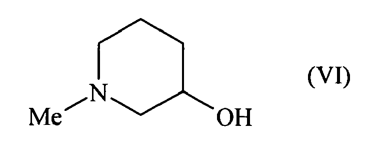 qui est soumis à l'action d'un dérivé de formule (IV),
les composés de formule (I/a) et (I/b), qui constituent l'ensemble des composés de formule (I), peuvent être ensuite purifiés selon une technique classique de séparation, que l'on transforme, si on le souhaite, en ses sels d'addition à un acide ou à une base pharmaceutiquement acceptable et dont les énantiomères peuvent être séparés sur colonne chirale, selon une technique classique de séparation.

8. Compositions pharmaceutiques contenant au moins un composé de formule (I) selon l'une quelconque des revendications 1 à 6 ou un de leurs sels d'addition avec un acide ou une base pharmaceutiquement acceptable en combinaison avec un ou plusieurs excipients pharmaceutiquement acceptables.

9. Compositions pharmaceutiques selon la revendication 8 utiles pour la fabrication de médicaments pour le traitement des déficits de mémoire liés au vieillissement cérébral et aux maladies neurodégénératives telles que la maladie d'Alzheimer, la maladie de Parkinson, la maladie de Pick, la maladie de Korsakoff, les démences vasculaires, les démences frontales et sous-corticales ainsi que la schizophrénie.

10. Utilisation des composés de formule (I) selon l'une quelconque des revendications 1 à 6 pour la fabrication de médicaments utiles pour le traitement des déficits de mémoire liés au vieillissement cérébral et aux maladies neurodégénératives telles que la maladie d'Alzheimer, la maladie de Parkinson, la maladie de Pick, la maladie de Korsakoff, les démences vasculaires, les démences frontales et sous-corticales ainsi que la schizophrénie.

11. Composés de formule (I) selon l'une quelconque des revendications 1 à 6 utiles pour le traitement des déficits de mémoire liés au vieillissement cérébral et aux maladies neurodégénératives telles que la maladie d'Alzheimer, la maladie de Parkinson, la maladie de Pick, la maladie de Korsakoff, les démences vasculaires, les démences frontales et sous-corticales ainsi que la schizophrénie.
